(19) **Europäisches Patentamt / European Patent Office / Office européen des brevets**

(11) **EP 1 787 265 B1**

(12) **FASCICULE DE BREVET EUROPEEN**

(45) Date de publication et mention
de la délivrance du brevet:
**23.12.2009 Bulletin 2009/52**

(51) Int Cl.:
***G06T 17/10*** *(2006.01)*

(21) Numéro de dépôt: **05805592.2**

(86) Numéro de dépôt international:
**PCT/FR2005/002200**

(22) Date de dépôt: **05.09.2005**

(87) Numéro de publication internationale:
**WO 2006/027490 (16.03.2006 Gazette 2006/11)**

(54) **DISPOSITIF POUR ETABLIR UNE REPRESENTATION TRIDIMENSIONNELLE COMPLETE D'UN MEMBRE D'UN PATIENT A PARTIR D'UN NOMBRE REDUIT DE MESURES PRISES SUR CE MEMBRE**

VORRICHTUNG ZUR ERSTELLUNG EINER VOLLSTÄNDIG DREIDIMENSIONALEN DARSTELLUNG EINES GLIEDS EINES PATIENTEN ANHAND EINER GERINGEN ANZAHL AN BESAGTEM GLIED VORGENOMMENER MESSUNGEN

DEVICE FOR CREATING A FULL THREE-DIMENSIONAL REPRESENTATION OF A LIMB OF A PATIENT FROM A REDUCED NUMBER OF MEASUREMENTS TAKEN FROM SAID LIMB

(84) Etats contractants désignés:
**AT BE BG CH CY CZ DE DK EE ES FI FR GB GR HU IE IS IT LI LT LU LV MC NL PL PT RO SE SI SK TR**

(30) Priorité: **06.09.2004 FR 0409384**

(43) Date de publication de la demande:
**23.05.2007 Bulletin 2007/21**

(73) Titulaire: **Laboratoires Innothera S.A.S.**
**94110 Arcueil (FR)**

(72) Inventeurs:
• **CROS, François**
**F-94200 Ivry Sur Seine (FR)**
• **FULLANA, José-Maria**
**F-75013 Paris (FR)**

(74) Mandataire: **Dupuis-Latour, Dominique et al**
**Bardehle Pagenberg Dost Altenburg Geissler**
**10, boulevard Haussmann**
**75009 Paris (FR)**

(56) Documents cités:
**WO-A-03/017206          US-A- 5 871 018**
**US-A1- 2004 068 337**

• **DATABASE INSPEC [Online] THE INSTITUTION OF ELECTRICAL ENGINEERS, STEVENAGE, GB; GEISEN G R ET AL: "Automatic detection, identification, and registration of anatomical landmarks from 3-D laser digitizer body segment scans" XP010215430 Database accession no. 5601294 & PROCEEDINGS OF 17TH INTERNATIONAL CONFERENCE OF THE ENGINEERING IN MEDICINE AND BIOLOGY SOCIETY 20-23 SEPT. 1995 MONTREAL, QUE., CANADA, vol. 1, 23 septembre 1995 (1995-09-23), pages 403-404 vol.1, XP010215430 1995 IEEE Engineering in Medicine and Biology 17th Annual Conference and 21 Canadian Medical and Biological Engineering Conference (Cat. No. 95CH35746) IEEE New York, NY, USA ISBN: 0-7803-2475-7**

**Description**

L'invention concerne la prise de données morphologiques d'un membre d'un patient.

Elle concerne plus précisément un dispositif pour établir une représentation tridimensionnelle complète d'un membre d'un patient à partir d'un nombre réduit de mesures prises sur celui-ci.

L'invention est applicable de façon particulièrement avantageuse à la sélection des orthèses de contention, c'est-à-dire des orthèses telles que bas, chaussettes, collants, etc. destinées à exercer un effort sur un segment de membre sous l'effet des forces de rappel des fibres élastiques, procurant ainsi une compression positive sur le membre. L'indidation habituelle de ce type d'orthèse est l'insuffisance veineuse des membres inférieurs.

L'une des difficultés, tout particulièrement avec les orthèses des classes les plus compressives, réside pour le prescripteur (généralement un pharmacien ou un orthopédiste) dans le choix d'une taille et d'une classe d'orthèse la mieux adaptée à la pathologie du patient, c'est-à-dire procurant sur toute l'étendue du membre une contention ni trop faible, ni trop forte.

En effet, sauf à faire réaliser un bas sur mesure, le praticien doit choisir une taille particulière dans des gammes dimensionnelles préexistantes, dont les pressions appliquées par les différents articles de ces gammes sont établies par rapport à un gabarit dont la forme et les dimensions sont normalisées ("modèle Hohenstein").

La délivrance d'une taille d'orthèse se fait, en pratique, par prise de mesure de la jambe du patient à différentes hauteurs, par exemple deux mesures périmétriques à la cheville et au mollet ou à la cuisse, et une mesure de hauteur sol-genou ou sol-entrejambe. À partir de ces mesures, le pharmacien ou l'orthopédiste détermine à partir d'un tableau ou d'une échelle la taille supposée être la mieux appropriée au patient.

Cette manière de procéder ne donne pas au prescripteur une véritable idée du profil de pression qui sera réellement appliqué à la jambe. Or la prescription d'une orthèse inappropriée peut se traduire localement par certaines zones de contention excessive ou, inversement, insuffisante.

Il existe certes des outils permettant, à partir d'une représentation tridi- , mensionnelle complète d'un membre sous forme d'un maillage de points, de simuler les valeurs des pressions de contention susceptible d'être exercées par une orthèse donnée, connaissant les caractéristiques dimensionnelles et rhéologiques de cette orthèse. Un tel outil est par exemple décrit dans le FR-A-2 852 421 (Laboratoires Innothéra), publié le 17 septembre 2004, pour, "*Dispositif d'aide à la sélection d'une orthèse de contention et à son adaptation à la morphologie d'un membre*" qui décrit des moyens, mis en oeuvre par logiciel, permettant à un praticien d'évaluer l'adaptation de telle ou telle dimension d'orthèse à la morphologie de la jambe d'un patient donné, de façon à pouvoir choisir en connaissance de cause celle qui est susceptible de procurer à ce patient l'effet thérapeutique optimal.

Cet outil implique cependant de disposer d'un fichier de données complet représentatif des caractéristiques morphologiques du membre du patient en question, sous forme d'un maillage de plusieurs centaines de points répartis à la surface du membre. L'obtention d'un tel fichier implique de réaliser une cartographie très précise du membre, par exemple au moyen d'une installation à pléthysmographe laser telle que celle décrite dans les FR-A-2 774 276 et FR-A-2 804 595 (Innothéra Topic International). Il s'agit cependant là d'un instrument de laboratoire qu'il n'est pas envisageable de mettre en oeuvre dans le cadre de l'officine d'un pharmacien ou du cabinet d'un orthopédiste. Il a certes été proposé divers dispositifs plus simples pour établir la cartographie complète d'un membre, mais leur coût unitaire et les conditions strictes pour leur mise en oeuvre n'ont pas permis d'en assurer une large diffusion auprès des prescripteurs potentiellement susceptibles de les utiliser.

WO03/017206 décrit une méthode de modélisation faciale dans laquelle la détection de points d'intérêts du visage permet l'adaptation d'un modèle 3D générique.

Un des buts de l'invention est de proposer un dispositif permettant, à partir d'un nombre réduit de mesures prises sur le membre (par exemple une mesure de hauteur et trois mesures de circonférence) d'établir une représentation tridimensionnelle du membre du patient sous forme d'un maillage complet de points, représentation qui puisse être soumise à tous les traitements informatiques souhaitables d'affichage, de simulation de contention et d'aide à la sélection d'une taille et d'un type d'orthèse appropriés - ces autres fonctions pouvant être aisément mises en oeuvre par un logiciel approprié chargé sur le micro-ordinateur du prescripteur.

Un autre but de l'invention est d'établir, à partir de ce dispositif et de relevés statistiques, une bibliothèque de morphotypes, c'est-à-dire de modèles de membres représentatifs. Cette bibliothèque de morphotypes pourra permettre, par exemple dans le cadre d'une étude clinique d'une population de patients, de voir si l'échelonnement existant de différentes tailles d'un produit est bien adapté à la majorité de cette population ou si, au contraire, une gamme de tailles différentes serait mieux appropriée pour couvrir les besoins du plus grand nombre de patients.

Pour atteindre les buts précités, l'invention propose un dispositif comprenant des moyens, en eux-mêmes connus, pour prendre sur le membre du patient des mesures comprenant une mesure de hauteur et une pluralité de mesures de circonférence prises à des cotes de mesure prédéterminées différentes.

De façon caractéristique de l'invention, il est prévu des moyens pour mémoriser un premier fichier de données comprenant les coordonnées d'un premier maillage de points représentatif de caractéristiques morphologiques d'un membre de référence, ces points étant répartis dans un espace tridimensionnel à la surface du membre de référence le long d'une succession de contours définis à différentes cotes successives de ce membre. Il est également prévu des moyens générateurs de morphologie, aptes à recalculer les coordonnées du premier maillage de points en fonction de ladite mesure de hauteur et desdites mesures de circonférence, de manière à produire et mémoriser un second fichier de données comprenant les coordonnées d'un second maillage de points représentatif de caractéristiques morphologiques du membre du patient.

Les coordonnées des points comprenant une coordonnée de cote et le premier fichier de données comprenant une donnée de hauteur de référence, les moyens générateurs de morphologie sont avantageusement des moyens aptes à recalculer la coordonnée de cote de chaque point du maillage, par application à cette coordonnée de cote d'une fonction de transformation paramétrée à la fois par ladite hauteur de référence et par ladite mesure de hauteur, notamment une fonction de redistribution proportionnelle paramétrée par le ratio entre la mesure de hauteur et la hauteur de référence.

Les coordonnées des points comprenant des coordonnées cartésiennes , ou polaires définissant la position du point sur un contour situé à une cote donnée, les moyens générateurs de morphologie sont avantageusement des moyens aptes à recalculer les coordonnées de position de chaque point du maillage, par application à ces coordonnées de position d'une fonction de transformation paramétrée par lesdites mesures de circonférence.

Dans ce cas, il est notamment possible de recalculer les coordonnées de position de chaque point du maillage situés à une cote égale auxdites cotes de mesure prédéterminées, en appliquant à ces coordonnées de position une fonction de transformation paramétrée par la déformation locale du contour mesurée à ladite cote par rapport au contour du membre de référence à cette même cote, cette déformation locale étant en particulier déterminée à partir de la différence entre la mesure de circonférence à ladite cote et la valeur de la circonférence du contour du membre de référence à cette même cote.

Quant aux points du maillage situés à une cote intermédiaire entre lesdites cotes de mesure prédéterminées, leurs coordonnées de position peuvent être recalculées interpolation entre les coordonnées de position recalculées de chaque point du maillage situé auxdites cotes de mesure prédéterminées, par exemple une interpolation linéaire ou une interpolation polynomiale d'ordre 2.

On va maintenant décrire un exemple de mise en oeuvre du dispositif de l'invention, en référence aux dessins annexés où les mêmes références numériques désignent d'une figure à l'autre des éléments identiques ou fonctionnellement semblables.

La figure 1 est une vue schématique montrant les différents moyens contribuant à la mise en oeuvre de l'invention.

La figure 2 illustre la modélisation de la jambe d'un patient.

La figure 3 illustre la transformation des données d'un membre de référence au membre du patient.

La figure 4 illustre, pour un exemple concret, l'effet des transformations successives permettant de passer du membre de référence au membre du patient, le membre étant vu de face sur la moitié supérieure de la figure et de trois-quarts arrière sur la moitié inférieure de cette même figure.

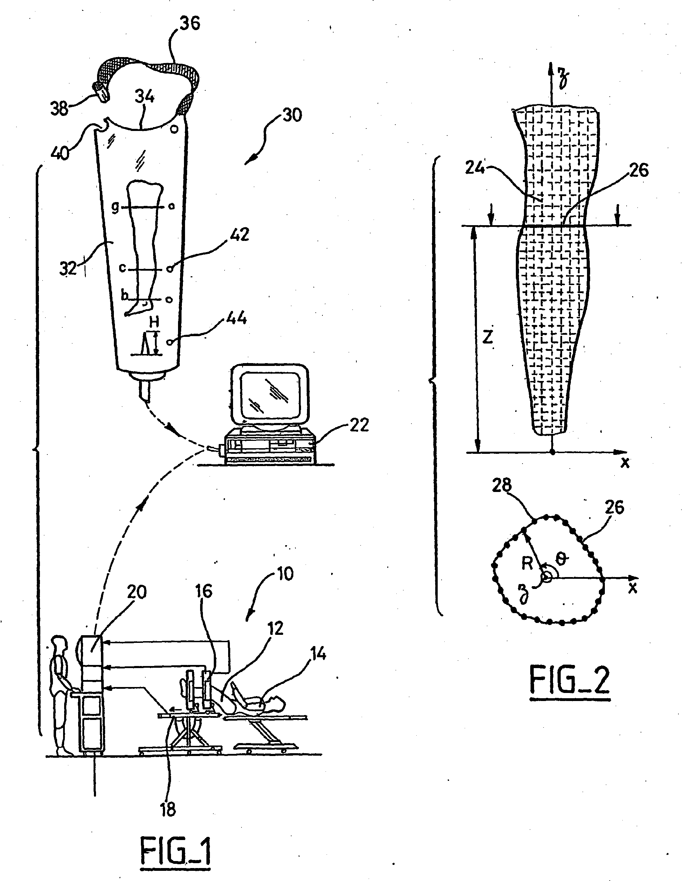

La figure 1 représente, de façon générale, les divers moyens permettant de mettre en oeuvre la présente invention.

La première étape est la constitution d'un fichier de données formant une représentation tridimensionnelle complète d'un membre de référence.

Cette représentation, qui est établie une fois pour toutes, peut être effectuée en laboratoire sur un sujet-type, ou bien sur un modèle de jambe de référence, par exemple une "jambe Hohenstein".

On peut par exemple utiliser à cet effet une installation 10 telle que celle présentée dans les FR-A-2 774 276 et FR-A-2 804 595 précités, qui décrivent un pléthysmographe laser permettant d'établir une cartographie très précise d'un membre 12 d'un sujet 14 le long de sections successives de ce membre. Le pléthysmographe 10 comporte une couronne de capteurs 16 permettant d'analyser par triangulation la forme d'une section de la jambe placée dans l'espace central de la couronne. Cette dernière peut être déplacée en translation le long d'un axe linéaire 18 sur toute la longueur du membre 12, par pas successifs, pour réitérer la prise de mesure pour différentes sections. Les signaux de mesure, ainsi que les positions de l'axe circulaire et de l'axe linéaire sont transmis à un dispositif 20 permettant de reconstituer à partir de ces informations une représentation tridimensionnelle de la jambe sous forme d'un fichier de données représentatives de la morphologique d'un membre de référence, fichier qui pourra être ultérieurement chargé dans le micro-ordinateur 22 d'un pharmacien ou orthopédiste prescripteur.

Cette représentation tridimensionnelle est illustrée figure 2. Elle se présente sous forme d'un fichier de données correspondant à un maillage 24 de points définis pour une série de sections 26 situées à des cotes Z successives. Ces données correspondent par exemple à la position de points 28 définis par leur cote Z et leurs coordonnées cartésiennes (x,y) ou polaires (r,$\theta$) par rapport à un référentiel Ox, Oz. L'origine du repère dans la direction z correspond à Z = 0 au sol, de manière à disposer d'une référence verticale absolue.

Une fois le fichier de données correspondant au membre de référence ainsi constitué, et chargé dans le micro-ordinateur du praticien prescripteur, celui-ci peut alors effectuer la prise des mesures sur les membres des différents patients qui le consultent.

Cette prise de mesure comprend par exemple quatre mesures, à savoir une mesure de hauteur H et trois mesures de circonférence b, c et g prises à des cotes de mesures prédéterminées : cheville, mollet et haut de cuisse, conformément au "standard Hohenstein" définissant la topographie des points de mensuration. Il est éventuellement possible de prévoir deux mesures supplémentaires d et f au genou et au bas de cuisse.

On peut avantageusement utiliser pour cette prise de mesure un dispositif tel que celui décrit dans le WO-A-00/44282 (Innothéra Topic International), qui est un dispositif 30 comprenant un boîtier 32 dont la forme est conçue pour qu'il soit aisément tenu dans une main, avec à l'une de ses extrémités une partie concave 34 destinée à être appliquée contre la jambe mesurée. Un ruban 36 est monté sur un enrouleur automatique qui comprend des moyens 38, 40 de verrouillage permettant de former avec ce ruban et la face concave un contour de circonférence variable autour du membre. La mesure de périmètre, définie par la dimension de cette boucle, est donnée par un codeur intégré au boîtier 32. Un bouton-poussoir 42 permet la validation de la mesure et sa mémorisation dans le micro-ordinateur 22. Il est également prévu un bouton-poussoir 44 correspondant à la mesure de la hauteur d'entrejambe, prise au moyen du ruban 36 ou par un autre moyen, par exemple un système télémétrique à ultrasons.

Les différentes mesures sont effectuées et validées par appui sur les boutons-poussoirs. Une fois cette opération effectuée, le micro-ordinateur 22 du prescripteur contient donc, d'une part, une représentation tridimensionnelle complète d'un membre de référence et, d'autre part, un nombre réduit de valeurs de mesures prises sur le membre d'un patient donné, au nombre de quatre ,(H, b, c, g) dans l'exemple décrit.

L'étape suivante consiste à opérer une transformation de la représentation tridimensionnelle du membre de référence, en fonction des mesures prises sur le membre du patient, pour aboutir à une nouvelle représentation tridimensionnelle complète qui sera, elle, représentative du membre du patient.

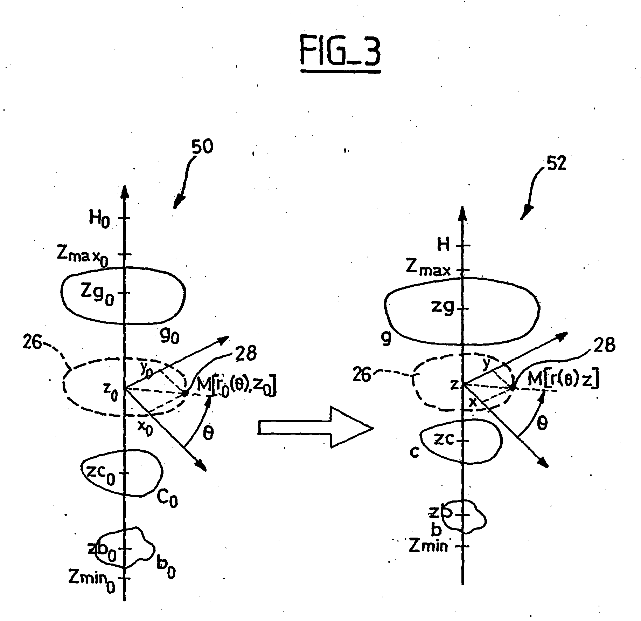

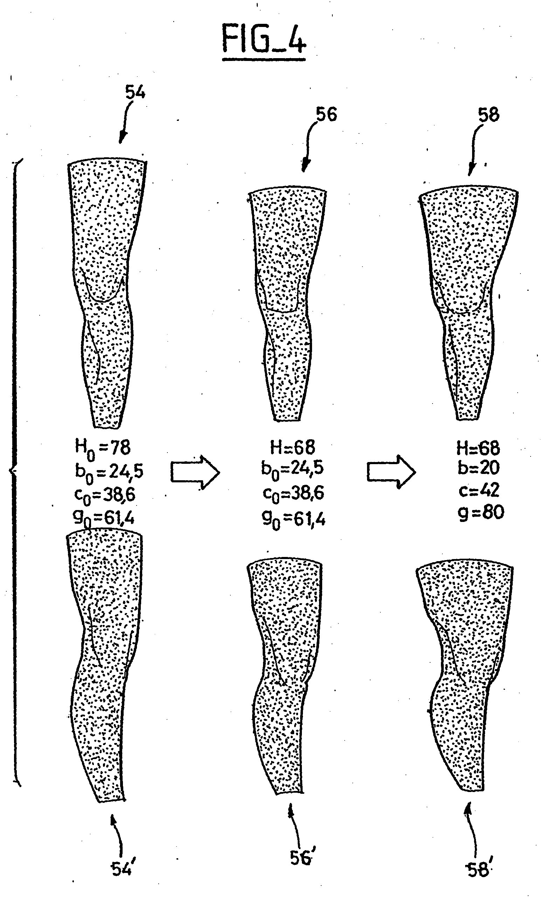

Cette transformation est illustrée sur les figures 3 et 4 (la figure 4 étant correspondant à un exemple particulier de mise en oeuvre). Cette transformation est effectuée en deux phases.

La première phase permet de prendre en compte la hauteur mesurée H pour modifier le maillage de points du membre de référence par redistribution des points de ce maillage sur la hauteur, de manière proportionnelle. Si l'on désigne par $H_o$ la valeur de hauteur du membre de référence et par $Z_{min}$ et $Z_{max}$ les cotes extrêmes du maillage de points, la transformation est opérée par un algorithme tel que le suivant :

Calcul du rapport de projection :

$$dh = H/H_o$$

Calcul des altitudes de début et de fin :

$$Z_{min} = dh\ Z_{min}$$

$$Z_{max} = dh\ Z_{max}$$

Projection de la morphologie :
Pour z = $Z_{min}$ à $Z_{max}$ :

Pour la coordonnée x :

Recherche des bornes locales $X_{sup}$ et $X_{inf}$ dans la morphologie initiale
Interpolation de x entre $X_{sup}$ et $X_{inf}$

Pour la coordonnée y :

Recherche des bornes locales $y_{sup}$ et $y_{inf}$ dans la morphologie initiale
Interpolation de y entre $y_{sup}$ et $y_{inf}$

La figure 4 illustre en 56 et 56' (respectivement de face et de trois-quarts arrière) le résultat de cette première redistribution opérée sur le membre de référence initial 54, 54' en fonction de la hauteur d'entrejambe H mesurée sur le patient.

La deuxième phase consiste à appliquer au maillage de points intermédiaire résultant de la transformation précédente les mesures de circonférence b, c et g.

Dans cette phase, la morphologie intermédiaire est modifiée à l'aide d'une fonction de déformation appelée f. Celle-ci est fabriquée à partir des déformations locales aux cotes des points de prise de mesure et généralisée aux autres cotes par interpolation linéaire ou polynomiale d'ordre 2, par un algorithme tel que le suivant :

Calcul des déformations locales :

$$f(g) = (g-g_o)/g_o$$

$$f(c) = (c-c_o)/c_o$$

$$f(b) = (b-b_o)/b_o$$

Calcul de la fonction de déformation :
f(z)
Calcul de la morphologie finale :

$$R(z) = [1+f(z)]\, r_o(z)$$

$$x = r(z)\cos\theta$$

$$x = r(z)\sin\theta$$

La figure 4 illustre en 58 et 58' (respectivement de face et de trois-quarts arrière) le résultat de cette seconde redistribution opérée sur la représentation intermédiaire 56, 56' en fonction des mesures de circonférence $\underline{b}$, $\underline{c}$ et $\underline{g}$.

On obtient ainsi un maillage complet donnant une représentation tridimensionnelle complète réaliste correspondant à un patient quelconque donné, simplement à partir d'un nombre très réduit de mesures prises sur celui-ci. Cette représentation tridimensionnelle complète pourra être ensuite aisément visualisée sur l'écran du micro-ordinateur du prescripteur, utilisée pour simuler les pressions de contention produites par tel ou tel type de bas, assister le prescripteur dans son choix d'un type et d'une taille de bas, etc.

Dans la description ci-dessus, la représentation du membre du patient a été réalisée à partir d'une unique morphologie de membre de référence. Cependant, pour prendre en compte la variabilité des patients éventuels, il est possible de disposer d'une pluralité de membres de référence différents correspondant à des morphologies-type telles que jambe très musclée, cuisse démusclée, oedème de cheville, etc. Il suffira au praticien de sélectionner comme membre de référence le morphotype le plus proche de celui du patient, le reste des opérations se déroulant de façon identique.

## Revendications

**1.** Un dispositif pour établir une représentation tridimensionnelle complète d'une partie du corps d'un patient à partir d'un nombre réduit de mesures prises sur ce membre, comprenant :

> - des moyens pour mémoriser un premier fichier de données comprenant les coordonnées ($x_o$, $y_o$, $z_o$) d'un premier maillage de partie du corps points (24) représentatif de caractéristiques morphologiques d'une partie du corps de référence, ces points (28) étant répartis dans un espace tridimensionnel à la surface de la partie du corps de référence le long d'une succession de contours (26) définis à différentes cotes successives (Z) de cette partie du corps

> **caractérisé en ce que** la partie du corps est un membre et **en ce qu'**il comprend en outre:

> - des moyens pour prendre des mesures sur le membre du patient, ces mesures comprenant une mesure de hauteur (H) et une pluralité de mesures de circonférence (b, c, g) prises à des cotes de mesure prédéterminées (zb, zc, zg) différentes,
> - des moyens générateurs de morphologie, aptes à recalculer les coordonnées du premier maillage de points en fonction de ladite mesure de hauteur (H) et desdites mesures de circonférence (b, c, g), de manière à produire et mémoriser un second fichier de données comprenant les coordonnées (x, y, z) d'un second maillage de points représentatif de caractéristiques morphologiques du membre du patient.

**2.** Le dispositif de la revendication 1 dans lequel, les coordonnées des points comprenant une coordonnée de cote (z) et le premier fichier de données comprenant une donnée de hauteur de référence ($H_o$), les moyens générateurs de morphologie sont des moyens aptes à recalculer la coordonnée de cote (z) de chaque point du maillage, par application à cette coordonnée de cote d'une fonction de transformation paramétrée à la fois par ladite hauteur de référence ($H_o$) et par ladite mesure de hauteur (H).

**3.** Le dispositif de la revendication 2, dans lequel les moyens générateurs de morphologie sont des moyens aptes à recalculer la coordonnée de cote (z) de chaque point du maillage, par application à cette coordonnée de cote d'une fonction de redistribution proportionnelle paramétrée par le ratio ($H/H_o$) entre la mesure de hauteur et la hauteur de référence.

**4.** Le dispositif de la revendication 1 dans lequel, les coordonnées des points comprenant des coordonnées cartésiennes (x, y) ou polaires (r, $\theta$) définissant la position du point sur un contour (26) situé à une cote donnée, les moyens générateurs de morphologie sont des moyens aptes à recalculer les coordonnées de position de chaque point du maillage, par application à ces coordonnées de position d'une fonction de transformation paramétrée par lesdites mesures de circonférence (b, c, g).

**5.** Le dispositif de la revendication 4, dans lequel les moyens générateurs de morphologie sont des moyens aptes à recalculer les coordonnées de position de chaque point du maillage situés à une cote égale auxdites cotes de mesure prédéterminées (zb, zc, zg), par application à ces coordonnées de position d'une fonction de transformation paramétrée par la déformation locale du contour mesurée à ladite cote par rapport au contour du membre de référence à cette même cote.

**6.** Le dispositif de la revendication 5, dans lequel les moyens générateurs de morphologie sont des moyens aptes à déterminer ladite déformation locale à partir de la différence (g-g$_o$ ; c-c$_o$ ; b-b$_o$) entre la mesure de circonférence (g ; c ; b) à ladite cote et la valeur de la circonférence (g$_o$ ; c$_o$ ; b$_o$) du contour du membre de référence à cette même cote.

**7.** Le dispositif de la revendication 5, dans lequel les moyens générateurs de morphologie sont des moyens aptes à recalculer les coordonnées de position de chaque point du maillage situés à une cote intermédiaire entre lesdites cotes de mesure prédéterminées par interpolation entre les coordonnées de position recalculées de chaque point du maillage situé auxdites cotes de mesure prédéterminées.

**8.** Le dispositif de la revendication 7, dans lequel ladite interpolation est une interpolation linéaire.

**9.** Le dispositif de la revendication 7, dans lequel- ladite interpolation est une interpolation polynomiale d'ordre 2.

**Claims**

**1.** A device for establishing a complete three-dimensional representation of a patient's body part from a reduced number of measurements taken on that limb, comprising :

- means for storing a first data file comprising the coordinates ($x_o$, $y_o$, $z_o$) of a first grid of points (24) that is representative of morphological characteristics of a reference body part, said points (28) being distributed in a three-dimensional space on the reference body part surface along a succession of contours (26) defined at different successive levels (Z) of said body part,

**characterized in that** the body part is a limb and furthermore comprises :

- means for taking measurements on patient's limb, said measurements comprising a height measurement (H) and a plurality of circumferential measurements (b, c, g) taken at different predetermined measurement levels (zb, zc, zg),
- morphology generating means, adapted to recalculate the coordinates of the first grid of points as a function of said height measurement (H) and of said circumferential measurements (b, c, g) so as to produce and store a second date file comprising the coordinates (x, y, z) of a second grid of points representative of morphological characteristics of patient's limb.

**2.** The device according to claim 1, wherein the coordinates of the points comprise a level coordinate (z) and the first data file comprises a reference height data (H$_o$), wherein the morphology generating means are means adapted to recalculate the level coordinate (z) of each point of the grid, by applying to said level coordinate a transformation function that is parameterized both by said reference height (H$_o$) and by said height measurement (H).

**3.** The device according to claim 2, wherein the morphology generating means are means adapted to recalculate the level coordinate (z) of each point of the grid, by applying to said level coordinate a proportional redistribution function that is parameterized by the ratio (H/H$_o$) between the height measurement and the reference height.

**4.** The device according to claim 1, wherein the coordinates of the points comprise cartesian (x, y) or polar (r, $\theta$) coordinates that define the position of a point on a contour (26) situated at a given level, wherein the morphology generating means are means adapted to recalculate the position coordinates of each point of the grid by applying to said position coordinates a transformation function that is parameterized by said circumferential measurements.

**5.** The device according to claim 4, wherein the morphology generating means are means adapted to recalculate the position coordinates of each point of the grid situated at a level equal to that of said predetermined measurement levels (zb, zc, zg) by applying to said position coordinates a transformation function parameterized by the local deformation of the contour measured at said level compared to the contour of the reference limb at that same level.

**6.** The device according to claim 5, wherein the morphology generating means are means adapted to determine said local deformation from the difference (g-g$_o$ ; c-c$_o$ ; b-b$_o$) between the circumferential measurement (g ; c ; b) at said level and the value of the circumference (go ; c$_o$ ; b$_o$) of the reference limb contour at that same level.

**7.** The device according to claim 5, wherein the morphology generating means are means adapted to recalculate the position coordinates of each point of the grid situated at an intermediary level between said predetermined measurement levels by an interpolation between the recalculated position coordinates of each point of the grid situated at said predetermined measurement levels.

**8.** The device according to claim 7, wherein said interpolation is a linear interpolation.

**9.** The device according to claim 7, wherein said interpolation is a polynomial interpolation of order 2.

**Patentansprüche**

**1.** Eine Vorrichtung zum Erstellen einer vollständigen dreidimensionalen Darstellung eines Körperteils eines Patienten basierend auf einer kleinen Zahl von auf genanntes Glied genommenen Messungen, umfassend :

> - Mittel zur Speicherung einer ersten Datendatei umfassend die Koordinaten $(x_o, y_o, z_o)$ eines ersten, für die morphologischen Charakteristiken eines Bezugskörperteils repräsentativen Netzes von Punkten (24), wobei genannte Punkte 28) in einem dreidimensionalen Raum auf der Oberfläche des Bezugskörperteils entlang einer Aufeinanderfolge von Konturen (26) auf verschiedenen aufeinanderfolgenden Höhen (Z) genanntes Körperteils verteilt sind,

> **dadurch gekennzeichnet, dass** der Körperteil ein Glied ist und ausserdem umfasst

> - Mittel, um Messungen auf dem Glied des Patienten zu nehmen , wobei genannte Messungen eine Höhemessung (H) und eine Vielzahl von auf verschiedenen vorbestimmten Messungshöhen (zb, zc, zg) genommenen Umfangmessungen (b, c, g) umfassen,
> - Morphologie erzeugende Mittel, die geeignet sind, die Koordinaten des ersten Netzes von Punkten in Abhängigkeit von genannter Höhemessung (H) und genannten Umfangmessungen (b, c, g) neuzukalkulieren, um eine zweite Datendatei zu erzeugen und speichern, die die Koordinaten (x, y, z) eines zweiten, für die morphologischen Charakteristiken des Gliedes eines Patienten repräsentativen Netzes von Punkten umfasst.

**2.** Die Vorrichtung gemäss Anspruch 1, wobei die Koordinaten des Punkts eine Höhenkoordinate (z) umfassen und die erste Datendatei einen Bezugshöhenwert $(H_o)$ umfasst, wobei die Morphologie erzeugenden Mittel Mittel sind, die geeignet sind, die Höhenkoordinate (z) jedes Punkts des Netzes durch Anlegen an diese Höhenkoordinate einer sowohl durch genannte Bezugshöhe (Ho) als auch durch genannte Höhemessung (H) parametrierten Transformationsfunktion neuzukalkulieren.

**3.** Die Vorrichtung gemäss Anspruch 2, in welcher die Morphologie erzeugenden Mittel Mittel sind, die geeignet sind, die Höhenkoordinate (z) jedes Punkts des Netzes durch Anlegen an diese Höhenkoordinate einer proportionalen durch das Verhältnis $(H/H_o)$ zwischen der Höhemessung und der Bezugshöhe parametrierten Neuverteilungsfunktion neuzukalkulieren.

**4.** Die Vorrichtung gemäss Anspruch 1, wobei die Koordinaten der Punkte die kartesischen (x, y) oder polaren (r, θ), die Lage des Punkts auf einer auf einer vorgegebenen Höhe liegende Kontur (26) bestimmenden Koordinaten umfassen, wobei die Morphologie erzeugenden Mittel Mittel sind, die geeignet sind, die Lagekoordinaten jedes Punkts des Netzes durch Anlegen an genannten Lagekoordinaten einer durch genannten Umfangsmessungen (b, c, g) parametrierten Transformationsfunktion neuzukalkulieren.

**5.** Die Vorrichtung gemäss Anspruch 4, in welcher die Morphologie erzeugenden Mittel Mittel sind, die geeignet sind, die Lagekoordinaten jedes auf einer genannten vorgegebenen Höhemessungen (zb, zc, zg) gleichen Höhe liegenden Punktes des Netzes durch Anlegen an genannte Lagekoordinaten einer durch die lokale Deformation der auf der genannten Höhe im Vergleich zur Kontur des Bezugsglieds auf derselben Höhe gemessenen Kontur parametrierte Transformationsfunktion neuzukalkulieren.

**6.** Die Vorrichtung gemäss Anspruch 5, in welcher die Morphologie erzeugenden Mittel Mittel sind, die geeignet sind, genannte lokale Deformation basierend auf dem Unterschied $(g-g_o \; ; \; c-c_o \; ; \; b-b_o)$ zwischen der Umfangmessung (g ; c ; b) auf genannter Höhe und dem Wert des Umfangs $(g_o \; ; c_o \; ; b_o)$ des Konturs des Bezugsglieds auf derselben Höhe zu bestimmen.

**7.** Die Vorrichtung gemäss Anspruch 5, in welcher die Morphologie erzeugenden Mittel Mittel sind, die geeignet sind, die Lagekoordinaten jedes auf einer Zwischenhöhe zwischen genannten Messungshöhen liegenden Punkts des Netzes neuzukalkulieren, wobei genannte Messungshöhen durch eine Interpolation zwischen den für jeden auf den vorgegebenen Messungshöhen liegenden Punkts des Netzes neukalkulierten Lagekoordinaten bestimmt sind.

**8.** Die Vorrichtung gemäss Anspruch 7, wobei genannte Interpolation eine lineare Interpolation ist.

**9.** Die Vorrichtung gemäss Anspruch 7, wobei genannte Interpolation eine polynomiale Interpolation der Ordnung 2 ist.

FIG_1

FIG_2

# FIG_3

# FIG_4

$$H_0 = 78$$
$$b_0 = 24,5$$
$$c_0 = 38,6$$
$$g_0 = 61,4$$

$$H = 68$$
$$b_0 = 24,5$$
$$c_0 = 38,6$$
$$g_0 = 61,4$$

$$H = 68$$
$$b = 20$$
$$c = 42$$
$$g = 80$$

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- FR 2852421 A
- FR 2774276 A
- FR 2804595 A
- WO 03017206 A
- WO 0044282 A